Europäisches Patentamt

European Patent Office

Office européen des brevets

(19)

(11) EP 0 605 453 B1

(12) FASCICULE DE BREVET EUROPEEN

(45) Date de publication et mention
de la délivrance du brevet:
08.01.1997 Bulletin 1997/02

(21) Numéro de dépôt: 92918239.2

(22) Date de dépôt: 07.08.1992

(51) Int Cl.6: C12P 19/44, C07H 3/02

(86) Numéro de dépôt international:
PCT/FR92/00782

(87) Numéro de publication internationale:
WO 93/04185 (04.03.1993 Gazette 1993/06)

(54) **Procédé de fabrication enzymatique d'alpha-glucosides et de leurs esters**

Verfahren zur enzymatischen Herstellung von Alpha-Glykosiden und deren Estern

Method for the enzymatic production of alpha-glucosides and esters thereof

(84) Etats contractants désignés:
AT BE CH DE DK ES FR GB GR IE IT LI LU MC NL
SE

(30) Priorité: 12.08.1991 FR 9110244

(43) Date de publication de la demande:
13.07.1994 Bulletin 1994/28

(73) Titulaire: ULICE S.A.
F-63200 Riom (FR)

(72) Inventeurs:
• PELENC, Vincent, Pascal
F-31000 Toulouse (FR)
• PAUL, François, Marie, Bernard
F-31400 Toulouse (FR)
• MONSAN, Pierre, Frédéric
F-31700 Mondonville (FR)

(74) Mandataire: Almond-Martin, Carol et al
Ernest Gutmann - Yves Plasseraud S.A.,
62 rue de Bonnel
69448 Lyon Cedex 03 (FR)

(56) Documents cités:
EP-A- 0 102 558      EP-A- 0 219 673
EP-A- 0 377 831      WO-A-90/09451
DE-A- 3 718 340      FR-A- 2 125 582
FR-A- 2 542 318      GB-A- 2 214 914

• CARBOHYDRATE RESEARCH vol. 87, 1980,
AMSTERDAM NL pages 27 - 34 K. ITANO
'Stereospecific preparation of monoglucosides
of optically active trans-1,2- cycloexanediols by
enzymic trans-D-glucosylation' cité dans la
demande
• BIOCHEMISTRY. vol. 9, no. 8, 1970, EASTON, PA
US pages 1833 - 1838 S.C. PAN 'Synthesis of
estriol 16 alpha-(alpha-D-glucoside) by enzymic
transglucosilation' cité dans la demande
• DATABASE WPIL Section Ch, Week 8902,
Derwent Publications Ltd., London, GB; Class
D17, AN 89011791

## Description

La présente invention concerne un procédé de fabrication enzymatique stéréospécifique d'$\alpha$-glucosides à partir de matières premières peu coûteuses et disponibles en grande quantité, notamment l'amidon et les maltodextrines ainsi que directement à partir de matières premières agricoles pouvant les contenir telles que farines ou semoules. L'invention concerne également un procédé d'estérification des $\alpha$-glucosides ainsi obtenus.

L'amidon est très largement distribué dans la nature. En effet, il constitue un aliment important pour les plantes et de grandes quantités sont mises en réserve par les organismes végétaux, afin d'entretenir la vie de la tige ou du tubercule pendant le repos hivernal et d'assurer le développement de l'embryon au cours de la germination.

Compte tenu de l'abondance de cette substance, de nombreuses tentatives de valorisation industrielle ont été faites, la plupart d'entre elles impliquant une réaction d'hydrolyse. L'amidon est hydrolysé par les acides dilues, l'hydrolyse acide totale fournissant du D-glucose. Des fragments de poids moléculaire plus élevé, les dextrines, sont obtenus par hydrolyse acide contrôlée et par l'action de la température. L'amidon peut également être hydrolysé par des enzymes (amylases).

Les produits d'hydrolyse de l'amidon peuvent servir dans la fabrication de glucosides. Ces composés, en particulier, les alkyl-glucosides, sont utiles comme surfactants et détergents non-ioniques biodégradables. Ils peuvent être utilisés comme agents émulsifiants dans des produits pharmaceutiques, cosmétiques et alimentaires.

La synthèse chimique des alkylglucosides à partir de glucose a déjà été décrite (par exemple, EP-A-0301298). Ce genre de procédé chimique peut comporter cependant un nombre très élevé d'étapes différentes et fournit toujours un mélange anomérique d'$\alpha/\beta$-alkylmono- et alkylpolyglucosides. Ces mélanges ne possédant pas de caractéristiques physico-chimiques précises présentent tous les inconvénients qui y sont attachés et notamment, absence de point de fusion précis, absence de régiosélectivité ou possibilité de réactions secondaires.

La fabrication enzymatique d'alkylglucosides à partir de maltose a également été proposée (S.C. Pan, Biochemistry, vol. 9, n° 8, 1970, pp 1833-1838 et Itano K. et al, Carbohydrate Research, 87, 1990, 27-34). Pan décrit la transglucosylation d'un alcool tel que le butanol avec, comme donneur de glucose, le maltose, en présence d'un surnageant de culture d'Aspergillus niger. L'identité de(s) l'enzyme(s) contenue(s) dans le surnageant et responsable(s) de la transglucosylation n'a pas été recherchée. La configuration anomérique ($\alpha$ ou $\beta$) des alkylglucosides obtenus n'a pas été vérifiée. Itano décrit la production enzymatique de l'$\alpha$-glucoside de cyclohexanediol utilisant, comme donneur de glucose, le maltose, et comme accepteur de glucose, le trans-1,2-cyclohexanediol. Cette réaction met en oeuvre un mélange grossier de différentes enzymes utilisées dans l'industrie agro-alimentaire, dont la plupart sont des hydrolases. Ces préparations contiennent, en outre, des enzymes non-identifiées.

Les rendements obtenus selon ces procédés enzymatiques sont faibles et ne permettent pas une synthèse des alkylglucosides à l'échelle industrielle. Par ailleurs, la configuration anomérique des produits obtenus est parfois imprévisible en raison des nombreuses enzymes présentes pendant la réaction et notamment en raison d'une activité $\beta$-glucosidase non identifiée.

Finalement, Fogarty et al (Biotechnology Letters, vol. 4, 1, 61-64, 1982) décrit la production d'$\alpha$-glycérolglucoside par la transglucosylation enzymatique de glycérol avec, comme donneur de glucose, l'$\alpha$-méthylglucoside. L'enzyme mise en oeuvre dans cette réaction est la transglucosidase purifiée d'A. niger. Dans les conditions décrites, le glycérol ne peut jouer le rôle d'accepteur de glucose qu'avec un nombre très limité de substrats. Ces résultats montrent que la transglucosidase, comme beaucoup de transférases, est capable d'agir sur un nombre relativement élevé d'accepteurs, mais qu'en revanche, le nombre de substrats ou donneurs dont elle peut se servir est très limité.

L'objet de la présente invention est de fournir une méthode de fabrication stéréospécifique d'$\alpha$-glucosides donnant lieu à des rendements élevés appropriés pour une exploitation à l'échelle industrielle et permettant d'utiliser, comme matières premières, de l'amidon ou des maltodextrines ainsi que des matières premières agricoles à forte teneur en amidon telles que des farines ou des semoules. La méthode recherchée doit être simple, directe et peu coûteuse. Un autre objet de l'invention est de fournir un procédé de traitement de matières amylacées brutes telles que l'amidon ou les maltodextrines, permettant la production de composés à haute valeur ajoutée, comprenant par exemple les esters d'$\alpha$-glucosides.

De manière surprenante, les inventeurs ont maintenant constaté que des $\alpha$-glucosides peuvent être fabriqués à l'échelle industrielle par $\alpha$-transglucosylation enzymatique, en utilisant comme substrats ou "donneurs de glucose" l'amidon ou des maltodextrines, et comme co-substrats ou "accepteurs de glucose", des alcools.

Il est tout a fait inattendu que des molécules polymériques, telles que l'amidon ou les $\alpha$-maltodextrines, puissent jouer le rôle de donneurs de glucose dans cette réaction. Comme indiqué ci-dessus, l'$\alpha$-transglucosidase peut effectuer le transfert d'un résidu glucosyl sur un nombre relativement élevé d'accepteurs (l'alcool dans le cas actuel), mais ne peut utiliser, comme substrats, que très peu de molécules.

L'invention concerne, plus particulièrement, un procédé de fabrication enzymatique d'$\alpha$-glucosides (également connus sous le nom d'$\alpha$-glucopyranosides), caractérisé par la mise en contact d'au moins un alcool dont le(s) seul(s) groupement(s) fonctionnel(s) est(sont) un(des) groupement(s) hydroxyl, avec de l'amidon, des maltodextrines ou du maltose se trouvant à une concentration d'au moins 100 g/l environ, en présence d'une préparation enzymatique purifiée présentant une activité d'α-transglucosylation et dépourvue d'activité β-glucosidase.

Les produits obtenus par ce procédé sont des α-glucosides constitués d'au moins une molécule de glucose et d'un alcool, dont la chaîne carbonée est de longueur et de structure variables selon le type d'alcool ayant participé à la réaction. Le produit est dépourvu de β-glucoside. La biosynthèse est effectuée dans des conditions de réaction peu agressives, à température moyenne (20°C à 70°C, par exemple entre 30°C et 60°C) et à pH compris entre 3 et 7, de préférence 4 et 6. La formation de produits de dégradation du glucose est ainsi évitée.

Dans le contexte de l'invention, une préparation enzymatique "présentant une activité d'α-transglucosylation" signifie n'importe quelle préparation enzymatique capable d'effectuer le transfert d'une molécule de glucose (à partir de matières premières constituées d'amidon ou contenant de l'amidon, ou constituées d'hydrolysats d'amidon ou contenant des hydrolysats d'amidon) sur l'alcool.

Par purifiée, il faut comprendre

- que la préparation enzymatique est dépourvue de toute autre activité enzymatique capable dans les conditions utilisées de catalyser la production de substances autres que les α-glucosides,
- que la préparation enzymatique est telle qu'elle évite la formation de produits secondaires,
- que la préparation enzymatique est dépourvue notamment d'activité β-glucosidase.

La préparation enzymatique peut être constituée d'une seule enzyme présentant une activité d'α-transglucosylation.

La préparation enzymatique peut également être constituée de plusieurs enzymes telles que soit chacune d'elles a une activité d'α-transglucosylation,
soit leurs activités enzymatiques mises en commun sont telles qu'elles confèrent à la susdite préparation enzymatique une activité d'α-transglucosylation.

Il peut s'agir d'une "authentique" α-transglucosidase, également connue comme "α-transglucosylase", c'est-à-dire une enzyme de type transférase. Ce type d'enzyme est normalement reconnu par sa capacité à synthétiser, en milieu dilué (par exemple, 100 g/L maltose) des oligosaccharides.

Comme α-transglucosidases, on peut citer les α-transglucosidases fongiques telles que celles provenant d'<u>Aspergillus niger</u>, <u>A. batatae</u>, <u>A. oryzae</u> ou <u>Talaromyces duponti</u>. Les α-transglucosidases <u>d'Aspergillus niger</u> et de <u>Talaromyces duponti</u> sont particulièrement préférées. Il est avantageux d'utiliser ces enzymes sous forme purifiée, la préparation étant ainsi dépourvue de toute activité β-glucosidase. La présence de β-glucosidase dans la préparation enzymatique empêche, en effet, la synthèse stéréospécifique des α-glucosides.

L'α-transglucosidase d'<u>Aspergillus niger</u>, disponible sous le nom commercial de TRANSGLUCOSIDASE-L (Amano Pharmaceutical Corporation Ltd, Japon), est normalement dépourvue de β-glucosidase et peut être utilisée dans le procédé de l'invention sans purification supplémentaire. En revanche, celle de <u>Talaromyces duponti</u> (également connue sous le nom de 1,4-α-D-glucan-6-α-D-glucosyl-transférase) est avantageusement soumise à une étape supplémentaire de purification, par échange d'ions, afin d'éliminer la β-glucosidase, mais présente l'avantage d'une grande thermostabilité. Cette enzyme est décrite dans la demande de brevet européen EP-A-0219673.

Il est également possible d'utiliser un mélange d'enzymes purifiées, par exemple les α-transglucosidases de différents types de champignons, à condition que d'autres enzymes ne soient pas présentes et que les différentes enzymes fonctionnent dans les mêmes conditions.

L'enzyme présentant une activité d'α-transglucosylation peut aussi être une hydrolase purifiée qui possède, comme propriété "marginale", la capacité d'effectuer des réactions de α-transglucosylation. Ce type d'enzyme peut généralement être reconnu par sa capacité de former, en milieu dilué (par exemple, 100 g/L maltose) du glucose. Comme exemples de ce type d'enzyme, on peut citer les hydrolases telles que l'α-glucosidase.

Bien que ces enzymes soient capables d'effectuer d'autres types de réactions que l'α-transglucosylation, le milieu réactionnel composé uniquement d'amidon, de maltodextrines ou de maltose, et de l'alcool favorise la formation des α-glucosides.

Comme substrats ou "donneurs de glucose", il est possible d'utiliser, selon l'invention, l'amidon, des maltodextrines ou du maltose ou un mélange de ces substances, ainsi que des matières premières agricoles pouvant contenir ces substances.

Lorsqu'on utilise le maltose comme substrat, il peut être présent sous forme de mélange avec d'autres substances provenant de matières amylacées, par exemple, lorsque le substrat est un hydrolysat partiel d'amidon, le maltose peut être présent avec des maltodextrines. Le maltose peut aussi être utilisé seul. Les rendements en α-glucoside obtenus à partir du maltose sont parfois moins importants que ceux obtenus à partir des autres types de substrats, et sont par exemple de l'ordre de 10 à 15 % (calculé comme dans l'exemple 1 ci-dessous).

Les maltodextrines constituent un substrat ou "donneur de glucose" particulièrement préféré selon le procédé de l'invention. Ce sont des hydrolysats partiels d'amidon, constitués d'un mélange d'oligosaccharides de masse molaire variable et présentant uniquement des liaisons $\alpha$-1,4 entre les unités de glucose. Les maltodextrines peuvent provenir d'une hydrolyse partielle acide de l'amidon ou d'un traitement thermique ou enzymatique de l'amidon. Ce type de produit est aussi disponible dans le commerce et, en raison de sa pureté, est particulièrement préféré lorsque les $\alpha$-glucosides obtenus sont destinés à être utilisés dans l'industrie pharmaceutique.

Selon une autre variante du procédé de l'invention, le substrat, ou "donneur de glucose", peut aussi être de l'amidon soluble. Ce produit, assimilable aux maltodextrines, est constitué d'amidon rendu soluble par préhydrolyse acide, par exemple selon le procédé de Lintner.

Selon un autre mode de réalisation particulièrement préféré de l'invention, le substrat est constitué d'amidon natif. Comme sources d'amidon natif, on peut citer les céréales, les tubercules et les légumineuses, ainsi que toute autre plante. Parmi les céréales préférées, on peut citer le blé, le maïs, l'orge, l'avoine, le riz, le seigle, le triticale (hybride de seigle et de blé) le sarrasin et le sorgho. Les tubercules préférées sont les pommes de terre et le manioc. Les pois et les féveroles sont source d'amidon de légumineuses. Les céréales peuvent être utilisées sous forme de grains entiers, de fractions de grains (résultant, par exemple, d'un traitement enzymatique, chimique, thermique ou mécanique du grain), ou de tout autre produit issu du broyage du grain, tel que la farine ou la semoule.

Lorsque le substrat est l'amidon natif, l'action de l'enzyme capable d'effectuer l'$\alpha$-transglucosylation est associée à l'action d'une hydrolase, afin de rendre l'amidon accessible à l'$\alpha$-transglucosylation. L'hydrolase libère, dans le milieu, des maltodextrines et du maltose à partir de l'amidon natif. Ces substrats sont alors utilisés comme donneurs de glucose par l'$\alpha$-transglucosidase qui glucosyle les alcools présents dans le milieu. De préférence, l'hydrolase est utilisée simultanément avec l'$\alpha$-transglucosidase.

Comme hydrolases, il est préférable d'utiliser des endo-amylases, par exemple l'$\alpha$-amylase (E.C : 3.2.1.1), provenant, par exemple, de champignons ou de <u>Bacillus licheniformis</u>. Les endo-amylases ont l'avantage d'effectuer des coupures "endo" dans la molécule de l'amidon, sans libérer de molécules de faible masse moléculaire (c'est-à-dire le glucose). En effet, les $\alpha$-transglucosidases ne sont pas capables d'utiliser le glucose comme substrat. La production de glucose par l'amylase empêcherait donc la fabrication ultérieure d'$\alpha$-glucosides. En ce qui concerne le maltose, bien qu'il puisse servir de substrat pour l'$\alpha$-transglucosidase, le rendement d'alkylglucoside ainsi obtenu est plus faible qu'avec les maltodextrines ou l'amidon. Le rapport entre le glucose transférable et le glucose inutilisé est plus élevé dans le cas d'une maltodextrine que dans le cas du maltose.

Une utilisation simultanée de l'amylase et de la $\alpha$-transglucosidase est préférée, une compétition pour les molécules solubles s'établissant entre les deux enzymes. Le déroulement simultané des deux procédés enzymatiques conduit ainsi à une amélioration du rendement.

L'efficacité de l'amylase sur l'amidon varie selon l'origine botanique de l'amidon et selon la nature de l'amylase. Les concentrations relatives du substrat, de l'enzyme et de l'alcool sont

- pour le substrat, d'au moins 100 g/l environ à environ 800g/l et notamment d'au moins 100g/l environ à environ 400g/l
- pour les alcools, d'environ 10g/l à environ 800g/l, et notamment d'environ 50g/l à environ 400g/l
- pour l'enzyme d'environ 5 U/ml à environ 1000 U/ml, et notamment d'environ 50 U/ml à environ 200 U/ml.

L'accepteur de glucose selon l'invention est un alcool de fonction simple, c'est-à-dire, un alcool qui ne porte pas de groupements fonctionnels autres que les groupements hydroxyl. On peut utiliser un mélange d'alcools différents. Tous les alcools capables de jouer le rôle d'accepteurs de glucose dans la réaction et qui n'inhibent pas l'activité de l'enzyme peuvent être utilisés.

Les alcools préférés sont les alcanols, les alcools éthyléniques, les alcools acétyléniques, les alcools cycliques ou les phénols. Les alcools acycliques sont particulièrement préférés, notamment les alcanols saturés ayant par exemple entre 2 et 24 atomes de carbone, et les polyols saturés. Selon le procédé de l'invention, l'alcool peut être un alcool monohydrique, ou peut être aussi un polyol, par exemple un diol ou un triol. Les diols sont particulièrement préférés tels que les propanediols et les butanediols. Les alcools primaires, secondaires et tertiaires peuvent servir dans la réaction de l'invention, les primaires et secondaires étant particulièrement avantageux.

Il peut s'agir d'un alcool miscible ou au moins partiellement soluble dans l'eau. Dans ce cas l'alcool présente avantageusement, une solubilité d'au moins 2.7 % v/v à 20°C. Dans la plupart des cas, ces alcools possèdent entre 1 et 6, et souvent entre 1 et 5 atomes de carbone.

Comme exemples d'alcools solubles préférés, on peut citer : l'isopropanol, le n-butanol, l'iso-butanol, l'iso-pentanol, le propanol, le pentane-diol, l'hexane-diol, le butene-1-ol-3, le butyne-1-ol-3, le cyclohexanol... ou un mélange d'au moins deux de ces alcools. La réaction se poursuit en milieu aqueux, l'alcool étant souvent utilisé à la limite de sa solubilité.

Lorsque l'alcool est partiellement soluble dans l'eau, il est possible d'effectuer le procédé de l'invention dans un milieu à deux phases, c'est-à-dire une phase aqueuse dans laquelle le ou les alcools sont dissous à une concentration inférieure à la limite de leur solubilité, et une phase organique en équilibre avec la phase aqueuse et composée de l'alcool saturé de tampon. L'utilisation d'un milieu à deux phases exerce un effet favorable sur le rendement en α-glucosides. Ce système est particulièrement avantageux lorsque l'α-glucoside obtenu par la réaction de α-transglucosylation peut, à partir d'une certaine concentration, constituer un substrat pour l'enzyme. Par exemple, l'α-butylglucoside peut jouer le rôle de substrat pour l'α-transglucosidase. Dans ce cas, la réaction se déroule dans la phase aqueuse, et l'α-glucoside est extrait depuis la phase aqueuse vers la phase organique, empêchant la destruction du glucoside par l'enzyme.

L'utilisation d'un alcool éthylénique tel que le butene-1-ol-3 conduit à la présence d'une liaison non-saturée dans le glucoside. Ceci permet l'utilisation ultérieure du glucoside dans des réactions de polymérisation ou la préparation de synthons chiraux (molécules intermédiaires) à usage pharmaceutique ou chimique.

Selon un mode de réalisation particulièrment avantageux, l'alcool soluble est un alcanol monohydrique ayant de 1 à 5 atomes de carbone. Ces alcools donnent lieu à des α-alkylglucosides, dont la chaîne alkyle a de 1 à 5 atomes de carbone.

Il est également possible d'utiliser des alcools insolubles dans l'eau, par exemple, des alcanols ayant plus de six atomes de carbone, en particulier entre 12 et 18 atomes de carbone ou encore des alcools gras (alcools aliphatiques primaires) tels que le dodécylalcool, le tétradécylalcool, l'hexadécylalcool, l'octadécylalcool, le décylalcool, l'undécylalcool et le tridécylalcool, etc... Comme alcools insolubles dans l'eau, on peut également citer les stéroïdes. L'utilisation des alcools gras est avantageusement accompagnée de l'utilisation d'alcools courts (1 à 6 atomes de carbone) partiellement solubles dans l'eau, qui jouent le rôle de solvants des alcools gras et d'accepteur de glucose.

Les alcools hydrophobes, tels que ceux cités ci-dessus, peuvent participer à la réaction d'α-transglucosylation enzymatique en utilisant un système à deux phases (c'est-à-dire, de l'eau et un solvant organique). L'alcool hydrophobe est donc dissous dans un solvant tel que le nitrobenzène, tandis que le substrat et l'enzyme sont dissous dans une solution aqueuse. Les deux solutions sont ensuite mélangées et agitées. L'α-transglucosylation a lieu à l'interphase. Il est également possible d'utiliser d'autres solvants organiques, par exemple l'acétone, à condition que l'activité de l'enzyme ne soit pas affectée.

En raison des propriétés tensioactives des α-alkylglucosides dont la chaîne alkyle présente entre 12 et 18 atomes de carbone, l'utilisation, comme alcool, des alcools gras est particulièrement préférée.

Les rendements en α-glucosides obtenus par le procédé de l'invention varient selon le donneur de glucose et selon la méthode d'extraction. Avec, par exemple, la maltodextrine ou l'amidon comme substrat et un système de solvant organique biphasique pour extraire le produit, les rendements peuvent atteindre 20 à 30 %. Le maltose donne des rendements de 10 à 15 %. Les rendements sont calculés de la façon suivante :

$$\frac{\alpha\text{-glucoside produit, moles}}{\alpha\text{-glucoside produit} + \text{glucose produit, moles}} \times 100$$

Les produits obtenus par le procédé de l'invention sont stéréospécifiques (α) et sont normalement des monoglucosides. Il est possible, en ajoutant par exemple au milieu réactionnel un α-alkylglucoside comme substrat "supplémentaire", de provoquer la formation de diglucosides, c'est-à-dire des maltosides. Ces maltosides présentent des propriétés très proches de celles des glucosides.

La stéréospécificité du produit peut être vérifiée en effectuant l'hydrolyse d'un échantillon avec une enzyme ne pouvant agir que sur les anomère α, par exemple l'α-glucosidase. La maltase est un exemple d'α-glucosidase appropriée pour cette vérification.

En raison de l'absence d'anomère β dans le produit de l'invention, certaines caractéristiques physiques, telles que le point de fusion et la solubilité du glucoside, sont définies très étroitement. Cette précision est avantageuse pour une utilisation dans l'industrie pharmaceutique, cosmétique et chimique en général.

De plus, l'enzyme utilisée lors de l'α-transglucosylation est à l'état purifié. Le produit de l'invention est donc dépourvu de contaminants issus de réactions enzymatiques secondaires. La nécessité d'effectuer de nombreuses étapes de purification des produits de la réaction est donc éliminée.

Les α-glucosides, en particulier les α-alkylglucosides, tels qu'obtenus par le procédé de l'invention, sont caractérisés par l'absence de contaminants normalement associés aux glucosides produits par voie chimique ou par voie enzymatique utilisant des mélanges d'enzymes. L'α-butylglucoside est un produit particulièrement préféré. Plus particulièrement, les α-glucosides tels qu'obtenus par le procédé de l'invention sont exempts d'anomères β et de tout contaminant issus de réactions enzymatiques secondaires.

L'extraction du produit du milieu réactionnel est donc d'autant plus facile qu'il ne contient, outre le produit, que l'excédent d'alcool, le donneur de glucose résiduel, et le glucose produit au cours de la réaction. L'extraction peut être faite dans un solvant organique approprié, choisi en fonction de la solubilité du produit. Selon un mode de réalisation préféré, l'extraction de l'α-glucoside, par exemple l'α-butylglucoside, s'effectue en continu au moyen d'une colonne liquide de solvant d'extraction, par exemple le butanol. Selon cette variante, le milieu réactionnel est passé d'abord sur une première colonne contenant la transglucosidase immobilisée, puis sur la colonne liquide d'extraction. La réaction d'alcoolyse et l'extraction du produit se font alors en continu. L'α-butylglucoside est récupéré sous forme de sirop après évaporation du butanol et le butanol est recyclé pour utilisation comme substrat. En utilisant ce type d'extraction biphasique, les rendements de produits atteignent 35 à 40 %.

Les glucosides produits selon le procédé de l'invention ont une multiplicité d'applications.

Les alkylglucosides, en particulier ceux dont la chaîne alkyle possède jusqu'à 6 atomes de carbone, par exemple l'α-butylglucoside, peuvent être utilisés comme additifs de détergents liquides, d'émulsions pour modifier la viscosité sans provoquer de séparation de phases ou comme co-tensioactif dans les micro-émulsions. En effet, la présence de groupes hydrophobes et hydrophiles dans ces molécules permet leur utilisation dans des émulsions, par exemple des crèmes cosmétiques et pharmaceutiques, pour modifier les propriétés d'une émulsion, sans la déstabiliser. Les glucosides de l'invention étant fabriqués par une biosynthèse à partir de matières naturelles, ils sont particulièrement adaptés aux applications cosmétiques et pharmaceutiques de ce type.

Les alkylglucosides, et en particulier l'α-butylglucoside, peuvent aussi être utilisés comme additifs dans l'industrie des plastiques, des caoutchoucs et PVC par exemple, comme assouplissants dans les résines mélaminiques. L'utilisation de l'α-butylglucoside empêche la résine d'avoir un caractère excessivement cassant. Ils peuvent également être utilisés comme additifs dans des compositions de nettoyants industriels et ménagers ou de produits d'entretien.

Les alkylglucosides, dont la chaîne alkyle possède au moins 8 atomes de carbone, sont utiles comme surfactants et détergents non-ioniques biodégradables, présentant des propriétés moussantes, lubrifiantes, émulsifiantes et hydratantes.

Puisqu'ils sont stéréospécifiques et sont obtenus par un procédé de synthèse enzymatique à partir de matières végétales, les produits tels qu'obtenus par le procédé de l'invention sont particulièrement appropriés à une utilisation pharmaceutique, cosmétique ou alimentaire, par exemple comme agents émulsifiants non toxiques et non irritants.

Les glucosides tels qu'obtenus par le procédé de l'invention constituent aussi une excellente matière première dans la fabrication de polyétherpolyols, par exemple, des mousses rigides à base de polyuréthane. Les produits ainsi obtenus ont une thermostabilité améliorée et d'excellentes propriétés physiques. Les glucosides, en particulier les alkylglucosides, peuvent également être utilisés comme polyols dans la fabrication de résines alkydes et de polyesters ou comme intermédiaire chimique pour la synthèse de monomères utilisables dans des réactions de polymérisation.

Les alkylglucosides tels qu'obtenus par le procédé de l'invention ayant des chaînes alkyles courtes ($C_1$ à $C_6$) peuvent servir comme matières premières dans la fabrication d'agents tensioactifs, par exemple en remplaçant la chaîne alkyle par une chaîne alkyle ayant au moins 8, et de préférence au moins 12, atomes de carbone. Ces alkylglucosides de chaîne alkyle longue présentent d'excellentes propriétés émulsifiantes, moussantes et sont biodégradables.

Un autre type d'agent tensioactif peut être produit par l'estérification, de préférence dans les positions $C_2$ et $C_6$, des alkylglucosides inférieurs de l'invention. Les émulsifiants ainsi obtenus ont des propriétés hydratantes et sont également biodégradables.

Selon une variante particulièrement préférée de l'invention, l'estérification des α-glucosides s'effectue par voie enzymatique en mettant en contact l'α-glucoside avec un acide gras ou avec un mélange de différents acides gras et une préparation enzymatique ayant une activité de lipase. Ce type de réaction a été décrit par Björkling et al (J. Chem. Soc., Chem. Comm., 1989, p 934-935). Le couplage de cette réaction d'estérification enzymatique avec la réaction d'alcoolyse de l'invention, permet la fabrication d'esters d'α-glucosides purs à partir d'amidon, de maltodextrine ou de maltose. L'estérification peut être effectuée après la réaction d'alcoolyse ou simultanément avec l'alcoolyse.

Selon cette variante de l'invention, l'α-glucoside utilisé est de préférence un alkylglucoside ayant entre 1 et 5 atomes de carbone ou un mélange de ces α-alkylglucosides. L'α-butylglucoside est particulièrement préféré. L'acide gras possède avantageusement entre 8 et 20 atomes de carbone. Les acides caprylique ($C_8$), caprique ($C_{10}$), laurique ($C_{12}$) et palmitique ($C_{16}$) sont particulièrement préférés. Il est également possible d'utiliser un mélange de plusieurs acides gras.

L'estérification est catalysée selon ce mode de réalisation par une préparation enzymatique ayant une activité de lipase, par exemple, une lipase provenant de Mucor miehei (par exemple le Lipozyme®), de Candida antarctica, de Humicola sp., de Candida cylindracea et de Pseudomonas sp.. Toute lipase capable d'effectuer la réaction sur l'acide gras choisi peut être utilisée. Il peut s'agir d'une préparation purifiée contenant une seule enzyme ou alternativement, un mélange de plusieurs enzymes présentant une activité de lipase. L'enzyme possédant une activité de lipase peut être immobilisée sur un support solide.

La réaction d'estérification est effectuée à une température située entre la température ambiante et 80°C environ, à condition qu'il s'agisse d'une température à laquelle l'acide gras choisi soit sous une forme liquide, et à laquelle l'enzyme soit stable. L'absence de solvant représente un avantage important sur le plan économique et environnemental.

Les produits obtenus par l'estérification enzymatique, sans solvant, des α-glucosides de l'invention sont des mélanges de mono- et de diesters. Les conditions opératoires peuvent être optimisées pour permettre l'obtention d'environ 80 % de monoesters. La réaction est régio-spécifique, l'estérification ayant d'abord lieu dans la position $C_6$, et ensuite

dans la position $C_2$, $C_3$ ou $C_4$ selon l'enzyme. Le diester peut être un mélange de 2,6⁻, 3,6⁻ et 4,6⁻ diesters. Dans ce cas, le produit comporte le monoester et un mélange de diesters. De préférence, le diester est un 2,6-diester. La production d'une proportion de diesters est inévitable puisqu'à partir d'une certaine concentration de monoesters l'enzyme reconnaît le monoester comme substrat, plutôt que l'$\alpha$-glucoside. Lorsqu'il n'est utilisé qu'un seul acide gras dans l'estérification, le diester porte deux groupes identiques. En revanche, s'il est utilisé un mélange de deux acides gras, le diester est un diester mixte, par exemple, le butyl-2-0-lauryl-6-0-stearyl-$\alpha$-D-glucopyranoside. Le choix des acides gras permet de faire varier les propriétés des esters produits, en particulier l'H.L.B. (hydrophilic/lipophilic balance).

Le produit de l'estérification peut être extrait du milieu réactionnel par un solvant organique approprié tel que le diéthyle oxyde ou, à l'échelle industrielle, l'hexane. Tout solvant dans lequel l'$\alpha$-glucoside et l'acide gras ne sont pas solubles, peut être utilisé. La récupération des esters est également possible sans addition de solvant organique. Le produit peut être utilisé tel quel, sans purification après élimination de l'enzyme immobilisé.

Les esters d'$\alpha$-glucosides ainsi produits sont exempts d'anomères $\beta$ et de produits de réactions secondaires normalement associés aux esters obtenus à partir de glucosides moins purs ou par voie chimique. Ces esters ont une multiplicité d'applications industrielles et peuvent être utilisés pour toute application conventionnelle d'esters de ce type. Les esters d'$\alpha$-glucosides et en particulier, les esters d'$\alpha$-alkylglucosides sont des tensioactifs non ioniques, non irritants, non toxiques et biodégradables, ayant des propriétés telles que des propriétés moussantes, émulsionnantes, solubilisantes, hydratantes, dispersantes, mouillantes ou lubrifiantes. Ils sont avantageusement utilisés comme additifs dans des produits cosmétiques et pharmaceutiques et agroalimentaires ou encore en chimie ou dans l'industrie des détergents comme tensioactifs ou précurseurs d'agents blanchissants. En agriculture, ils peuvent être utilisés comme adjuvant inerte pour augmenter l'efficacité de produits phytosanitaires, notamment des herbicides, fongicides, insecticides et pour le traitement des cultures et des produits agricoles en général.

Les figures montrent différents aspects de l'invention, notamment :

- la figure 1 montre la production de glucose et d'$\alpha$-butylglucoside à partir de maltodextrines à l'aide de la $\alpha$-transglucosidase de <u>Talaromyces duponti</u>.
- la figure 2 illustre des chromatogrammes HPLC du milieu de synthèse d'$\alpha$-butylglucoside avec la transglucosidase d'<u>Aspergillus niger</u> à partir d'amidon insoluble : milieu à une phase liquide.

      a) durée de réaction : 10 mn
      b) durée de réaction : 23 heures
      c) mélange $\alpha$-$\beta$-butylglucoside.

- la figure 3 illustre des chromatogrammes HPLC du milieu de synthèse d'$\alpha$-butylglucoside avec la transglucosidase d'<u>Aspergillus niger</u> à partir d'amidon insoluble : milieu à deux phases liquides :

      a) durée de réaction : 10 mn
      b) durée de réaction : 7 heures

## **EXEMPLES**

**Exemple 1** : Production d'$\alpha$-butylglucoside à partir de substrats amylacés solubles en présence de butanol à l'aide de l'$\alpha$-transglucosidase de <u>Talaromyces duponti</u> :

a) <u>Dosage de l'activité $\alpha$-transglucosidase</u> :
Le dosage de l'activité $\alpha$-transglucosidase est effectué à 60°C dans le milieu suivant :

- maltose :     100 g/l
- Tampon acétate de sodium pH 4,5 :    50 mM
- $\alpha$-Transglucosidase :    1 U/ml

Le milieu est analysé après 15 et 24 heures de réaction par une technique HPLC (colonne DIONEX Carbopack, détection ampérométrique) permettant la séparation et la détection du panose formé au cours de la réaction.
1 unité de $\alpha$-Transglucosidase (U) est la quantité d'enzyme qui catalyse la production d'1 micromole de panose (6-0-$\alpha$-D-glucopyranosyl-maltose) par heure dans les conditions présentées ci-dessus.
b) <u>Production d'$\alpha$-butylglucoside</u> :
La production d'$\alpha$-butylglucoside est effectuée dans le milieu aqueux de composition suivante :

- Butanol : 9 % (v/v)
- Tampon acétate de sodium pH 4.5 : 50 mM
- α-Transglucosidase de Talaromyces duponti : 100 U/ml
- Substrat : 100 g/l

Ce milieu est incubé à la température de 50°C pendant 70 heures. Des prélèvements sont effectués, dilués (1/10) dans l'eau, chauffés à 90°C pour arrêter la réaction, puis analysés par chromatographie liquide à haute performance (CLHP) sur une colonne de résine échangeuse d'ions sous forme calcium. L'éluant est l'eau ultrapure.

Dans ces conditions d'analyse, l'α-butylglucoside est séparé du β-butylglucoside et des mono- et oligosaccharides. L'aire du pic correspondant permet de calculer la concentration d'α-butylglucoside dans le milieu réactionnel et le rendement de transfert de glucose défini de la façon suivante :

$$\frac{\alpha\text{-butylglucoside, mM}}{(\alpha\text{-butylglucoside + glucose}) \text{, mM}}$$

Les résultats sont présentés dans le tableau 1 :

Tableau 1 :

| α-butylglucoside produit à partir de substrats solubles et rendements de transfert après 70 heures de réaction. | | |
|---|---|---|
| Substrat | α-Butylglucoside, en mM | Rendement, en % |
| Maltose | 21 | 10 |
| Maltopentaose | 50 | 15 |
| Maltodextrines | 48 | 19 |
| Amidon soluble | 40 | 15 |

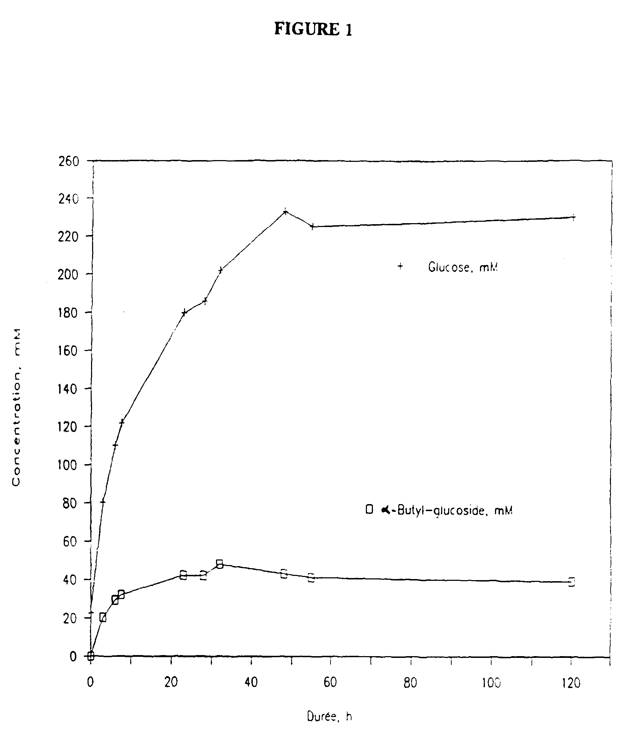

La production d'α-butylglucoside et de glucose a été suivie au cours de la réaction. L'évolution de la concentration de ces deux produits est indiquée sur la figure 1.

La production d'α-butylglucoside a lieu avec les quatre substrats amylacés testés dans cet exemple. Avec les maltodextrines, la concentration d'α-butylglucoside est supérieure à 10 g/l.

**Exemple 2** : Production d'α-butylglucoside à partir d'amidon insoluble en présence de butanol à l'aide de la α-transglucosidase de Talaromyces duponti.

Le milieu réactionnel est constitué, comme dans l'exemple 1. Le substrat est ici l'amidon insoluble. On rajoute dans le milieu de l'α-amylase de Bacillus licheniformis (5 U/ml), afin de permettre l'utilisation des substrats insolubles.

Les prélèvements du milieu réactionnel sont traités comme dans l'exemple 1, mais centrifugés et filtrés avant l'injection HPLC.

Les résultats sont présentés dans le tableau 2 :

Tableau 2 :

| α-butylglucoside produit à partir d'amidon insoluble et rendements de transfert après 70 heures de réaction. | | |
|---|---|---|
| Substrat | α-butylglucoside, en mM | Rendement, en % |
| Amidon insoluble + α-amylase | 28 | 16 |

L'amidon insoluble peut aussi être utilisé comme substrat de la réaction.

**Exemple 3** : Production d'α-butylglucoside à partir de substrats amylacés insolubles dans un milieu à deux phases liquides à l'aide de l'α-transglucosidase de Talaromyces duponti.

Le milieu réactionnel est constitué de deux phases liquides de composition suivante :

Phase aqueuse (volume : 0,5 ml) :

- Butanol : 9 % (v/v)
- Tampon acétate de sodium pH 4.5 : 50 mM
- α-Transglucosidase de Talaromyces duponti : 100 U/ml
- α-amylase : 5 à 500 U/ml
- Substrat : 100 g/L

Phase organique (volume : 0,5 ou 2 ml)

- Butanol saturé de tampon acétate de sodium 50 mM, pH 4.5.

Les milieux réactionnels sont vigoureusement agités avant chaque prélèvement, puis traités comme dans l'exemple 2.

Plusieurs milieux ont été expérimentés avec deux volumes réactionnels et deux préparations d'α-amylase. L'influence de la teneur en α-amylase du milieu a aussi été étudiée.

Les résultats d'analyse HPLC des milieux réactionnels sont présentés dans les tableaux 3 et 4 :

Tableau 3 :

| α-butylglucoside produit et rendements de transfert après 70 heures de réaction en milieu à deux phases liquides (α-amylase de Bacillus licheniformis, 5 U/ml) | | |
|---|---|---|
| Substrat volume réactionnel, ml | α-butylglucoside, mM | Rendement, % |
| Amidon insoluble 1 | 46 | 23 |
| Amidon insoluble 2,5 | 25 | 23 |

Tableau 4 :

| α-butylglucoside produit et rendements de transfert à partir de farine de maïs à l'aide de la a-transglucosidase de Talaromyces duponti en milieu à deux phase liquides (volume réactionnel : 1ml) | | |
|---|---|---|
| Origine et activité de l'α-amylase | α-butylglucoside, mM | Rendement, % |
| B. licheniformis 5 U/ml | 14 | 24 |
| B. licheniformis 50 U/ml | 15 | 19 |
| B. licheniformis 500 U/ml | 9 | 14 |
| A. niger 5 U/ml | 10 | 14 |
| A. niger 50 U/ml | 12 | 17 |
| A. niger 500 U/ml | 9 | 19 |

L'utilisation de farine de céréales brute est tout à fait possible pour la synthèse d'α-butylglucoside à l'aide de l'α-transglucosidase de Talaromyces duponti.

L'utilisation d'un milieu à deux phases liquides permet d'améliorer la production d'α-butylglucoside. Dans le tableau 3, on voit que 46 μmoles d'α-butylglucoside ont été synthétisées dans le milieu de volume 1 ml, alors que 62.5 μmoles ont été synthétisées dans le milieu de 2.5 ml à partir de la même quantité de substrat et d'enzymes.

**Exemple 4** : Production d'α-alkylglucosides à partir de substrats amylacés solubles à l'aide de l'α-transglucosidase de Talaromyces duponti en présence d'un mélange d'alcools.

Trois milieux ont été constitués de la façon suivante :

- Maltodextrines :          100 g/l
- a-Transglucosidase de Talaromyces duponti :          100 U/ml
- Tampon acétate de sodium pH 4.5 :          50 mM

Les trois milieux contiennent un mélange de mono-alcools :

|  | Essai 1 | Essai 2 | Essai 3 |
|---|---|---|---|
| Butanol | 9 % | 45 % | - |
| Pentanol | - | - | 25 % |
| Isopropanol | - | 25 % | 45 % |
| Eau | 91 % | 30 % | 30 % |

Les milieux ont été incubés à 50°C pendant 120 heures puis analysés selon la méthode décrite dans l'exemple 1. Les résultats d'analyse sont indiqués dans le tableau 5 :

Tableau 5 :

| Alkylglucosides produits en présence de mélanges d'alcools (volume réactionnel : 1ml) | | | |
|---|---|---|---|
|  | Essai 1 | Essai 2 | Essai 3 |
| α-butylglucoside, g/L | 10 | 5 | - |
| Isopropyl-glucoside, g/L | - | 2 | 6 |
| Pentyl-glucoside, g/L | - | - | 2 |

**Exemple 5** : Production d'α-butylglucoside à partir d'amidon insoluble à l'aide de l'α-transglucosidase d'Aspergillus niger

Deux milieux ont été constitués puis incubés selon le protocole indiqué dans l'exemple 3.

**Milieu à une phase aqueuse (volume : 20 ml)**

- Butanol :          9 % (v/v)
- Tampon acétate de sodium pH 5.5 :          50 mM
- α-Transglucosidase d'Aspergillus niger :          100 U/ml
- α-amylase de Bacillus licheniformis :          5 U/ml
- Amidon insoluble :          100 g/l

**Milieu à deux phases liquides (volume : 40 ml)**

Phase aqueuse (volume : 20 ml) :

- Butanol :          9 %
- Tampon acétate de sodium pH 5.5 :          50 mM
- α-Transglucosidase d'Aspergillus niger :          100 U/ml
- α-amylase de Bacillus licheniformis :          5 U/ml
- Amidon insoluble :          100 g/l

Phase organique (volume : 20 ml) :

- Butanol saturé de tampon d'acétate de sodium 50 mM, pH 5.5

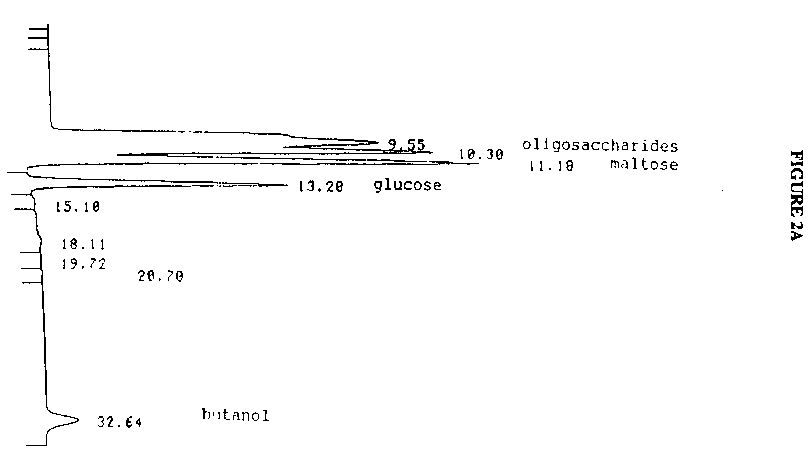

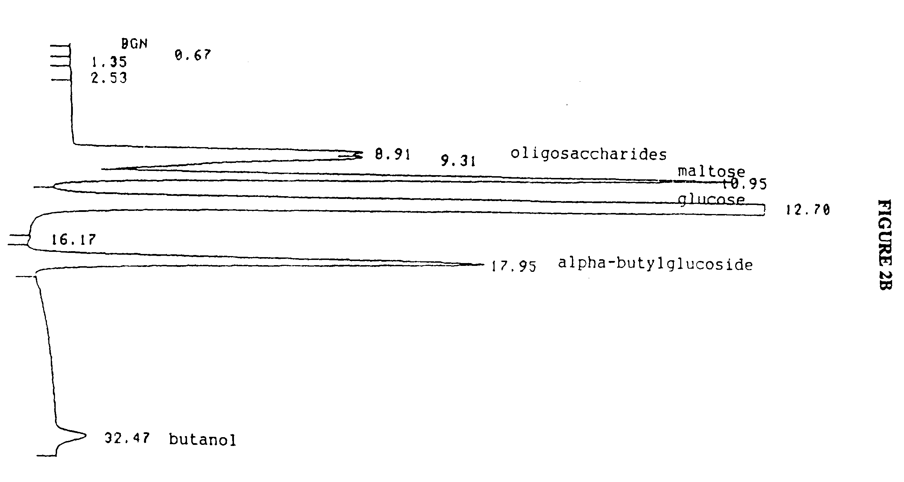

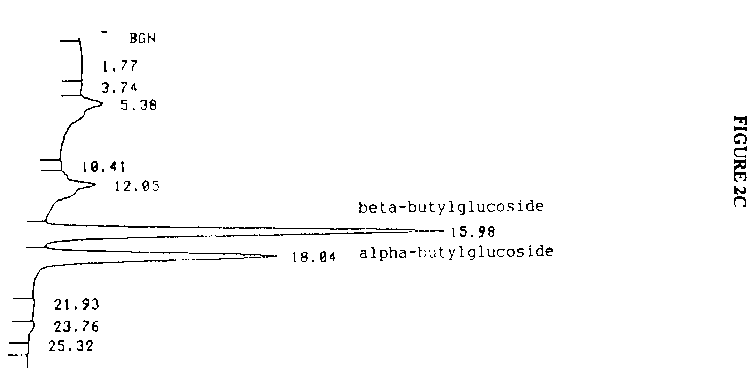

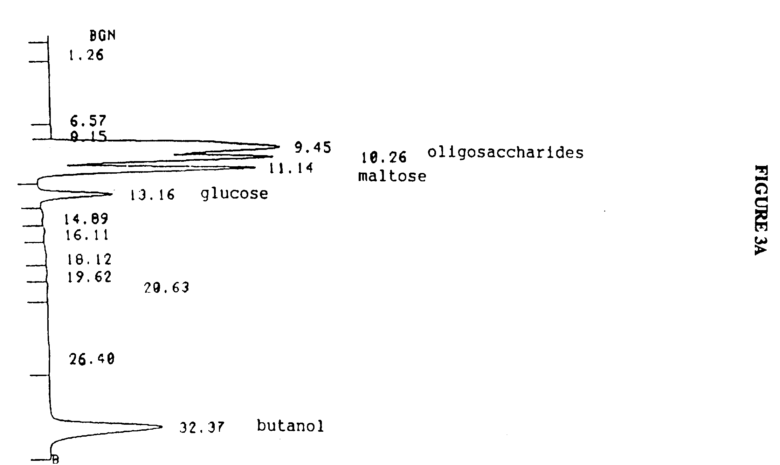

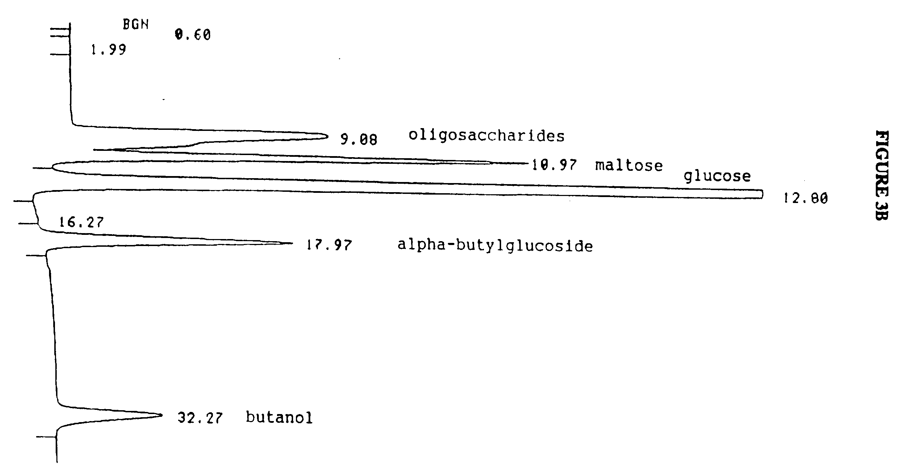

Ces milieux ont été analysés comme dans l'exemple 3. Les chromatogrammes obtenus sont présentés dans les figures 2 et 3. La concentration d'α-butylglucoside est de 12 g/l (51 mM) dans le milieu à une phase liquide et de 7 g/L (30 mM) dans le milieu à deux phases liquides.

L'α-Transglucosidase d'Aspergillus niger est tout à fait efficace pour la synthèse d'α-alkylglucoside.

**Exemple 6** : Production d'esters palmitiques d'α-butylglucoside par estérification enzymatique d'α-butylglucoside :

Un milieu a été constitué de la façon suivante :

- acide palmitique (PROLABO, France) :          10,85 g
- α-D-butylglucoside :          10 g

L'α-D-butylglucoside a été préparé selon la méthode de l'exemple 1.

Le mélange est porté à 75°C, température de fusion de l'acide palmitique. Après homogénéisation, on ajoute 1 g de Lipozyme®, lipase de <u>Mucor miehei</u> immobilisée sur support solide (NOVO INDUSTRI, Danemark).

Après 3 jours d'incubation, le milieu réactionnel est dilué avec 210 ml de diéthyle oxyde (SDS, France), puis filtré de façon à éliminer le Lipozyme®. Après filtration, on ajoute 50 ml de soude (NaOH 0,02 N), de façon à solubiliser dans la phase aqueuse l'acide palmitique résiduel sous la forme d'un sel de sodium, ainsi que l'α-butylglucoside résiduel.

La phase organique (diéthyle oxyde) contenant les esters de l'acide palmitique est évaporée sous vide, de façon à éliminer toute trace de solvant résiduel.

On obtient finalement 16,5 g d'esters (mélange de monoesters et de diesters d'α-D-butylglucopyranoside: butyl-6-0-palmityl-α,D-glycopyranoside et butyl-2,6-di-0-palmityl-α,D-glucopyranoside). La caractérisation des produits de la réaction a été faite par C.C.M. et par R.M.N. du proton et du $^{13}$C.

**Exemple 7** : Production d'esters lauriques d'α-butylglucoside par estérification enzymatique d'α-butylglucoside :
Un milieu a été constitué de la façon suivante :

- acide laurique (MERCK, Allemagne) :  8,5 g
- α-D-butylglucoside :  10 g

L'α-D-butylglucoside a été préparé selon la méthode de l'exemple 1.

Le mélange est porté à 45°C, température de fusion de l'acide laurique. Après homogénéisation, on ajoute 1 g de Lipozyme®, lipase de <u>Mucor miehei</u> immobilisée sur support solide (NOVO INDUSTRI, Danemark).

Après 3 jours d'incubation, le milieu réactionnel est dilué avec 210 ml de diéthyle oxyde (SDS, France), puis filtré de façon à éliminer le Lipozyme®. Après filtration, on ajoute 50 ml de soude (NaOH 0,02 N), de façon à solubiliser dans la phase aqueuse l'acide laurique résiduel sous la forme d'un sel de sodium, ainsi que l'α-butylglucoside résiduel.

La phase organique (diéthyle oxyde) contenant les esters de l'acide laurique est évaporée sous vide, de façon à éliminer toute trace de solvant résiduel.

On obtient finalement 15 g d'esters (mélange de monoesters et de diesters d'α-D-butylglucopyranoside: butyl-6-0-lauryl-α,D-glucopyranoside et butyl-2,6-di-0-lauryl-α,D-glucopyranoside. La caractérisation des produits de la réaction a été faite par C.C.M. et par R.M.N. du proton et du $^{13}$C.

**Exemple 8** : Production d'esters stéariques d'α-butylglucoside :
La méthode de l'exemple 7 a été répétée en utilisant :

- acide stéarique :  1,2 g
- α-D-butylglucoside :  1 g

Le mélange est porté à 78°C et on ajoute 0,15 g de Lipozyme®.

Le produit a été caractérisé par $^{13}$C-R.M.N. Il s'agissait d'un mélange principalement constitué de butyl-6-0-stéaryl-α,D-glucopyranoside (environ 80 %). de butyl-2,6-di-0-stéaryl-α,D-glucopyranoside (environ 20 %).

**Revendications**

1. Procédé de fabrication enzymatique d'α-glucosides, caractérisé par la mise en contact d'au moins un alcool dont le(s) seul(s) groupement(s) fonctionnel(s) est(sont) un(des) groupement(s) hydroxyl, avec de l'amidon, des maltodextrines ou du maltose, se trouvant à une concentration d'au moins 100 g/l environ, en présence d'une préparation enzymatique purifiée présentant une activité d'α-transglucosylation et dépourvue d'activité β-glucosidase.

2. Procédé selon la revendication 1, caractérisé en ce que la préparation enzymatique est constituée d'une enzyme présentant une activité d'α-transglucosylation ou de plusieurs enzymes telle que chacune ait une propriété d'α-transglucosylation ou telle que leurs activités enzymatiques mises en commun confèrent à la préparation enzymatique une activité d'α-transglucosylation.

3. Procédé selon l'une quelconque des revendications 1 ou 2 caractérisé en ce que le ou les enzymes sont des α-transglucosidases, de préférence provenant d'un champignon tel que <u>Talaromyces duponti</u>, <u>Aspergillus niger</u>, <u>Aspergillus oryzae</u> ou <u>Aspergillus batatae</u>.

4. Procédé selon l'une quelconque des revendications 1 à 3 caractérisé en ce que l'amidon est sous forme d'amidon natif et en ce que l'action de la préparation enzymatique capable d'effectuer l'α-transglucosylation est associée à l'action d'une hydrolase telle qu'une endo-amylase, par exemple l'α-amylase.

5. Procédé selon la revendication 4, caractérisé en ce que les deux réactions enzymatiques se déroulent simultanément par exemple, par l'utilisation simultanée d'une endo-amylase et d'une α-transglucosidase.

6. Procédé selon la revendication 4 ou 5 caractérisé en ce que l'amidon natif provient de céréales telles que le blé, le maïs, l'orge, l'avoine, le riz, le seigle, le sarrasin, le sorgho et le triticale, ou de tubercules telles que la pomme de terre ou le manioc, ou de légumineuses telles que les pois ou les féveroles, ou de toute autre plante.

7. Procédé selon la revendication 6 caractérisé en ce que les céréales sont sous forme de grains entiers, ou de fractions de grains, résultant par exemple d'un traitement enzymatique, chimique, thermique ou mécanique du grain, ou encore de tout produit issu du broyage du grain tel que la farine ou la semoule.

8. Procédé selon l'une quelconque des revendications 1 à 3 caractérisé en ce que l'amidon est sous forme d'amidon soluble, résultant par exemple d'une préhydrolyse acide ou enzymatique.

9. Procédé selon l'une quelconque des revendications précédentes caractérisé en ce que l'alcool est un alcanol, un alcool éthylénique, un alcool acétylénique, un alcool cyclique ou un phénol.

10. Procédé selon la revendication 9 caractérisé en ce que l'alcool est un alcool simple ou un polyol, de préférence un diol.

11. Procédé selon l'une quelconque des revendications 9 ou 10 caractérisé en ce que l'alcool possède de 1 à 6 atomes de carbone, est au moins partiellement soluble dans l'eau et présente, de préférence, une solubilité d'au moins 2,7 % (v/v) à 20°C.

12. Procédé selon l'une quelconque des revendications 9 à 11 caractérisé en ce que l'alcool est choisi parmi le groupe suivant : iso-propanol, n-butanol, iso-butanol, tert-butanol, iso-pentanol, hexane-diol, butène-1-ol-3, butyne-1-ol-3 ou un mélange d'au moins deux de ces alcools.

13. Procédé selon la revendication 9 caractérisé en ce que l'alcool est un alcanol possédant de 1 à 5, et en particulier 4, atomes de carbone, le produit du procédé étant alors un α-alkylglucoside, en particulier l'α-butylglucoside.

14. Procédé selon la revendication 9 caractérisé en ce que l'alcool est un alcool aliphatique primaire, par exemple, un alcool gras ayant entre 12 et 18 atomes de carbone.

15. Procédé selon l'une quelconque des revendications 1 à 14 caractérisé en ce qu'il est effectué entre 20 et 70°C, de préférence entre 20°C et 50°C, et à pH compris entre 3 et 7, de préférence entre 4 et 6.

16. Procédé de fabrication enzymatique d'esters d'α-glucosides à partir d'amidon, de maltodextrines ou de maltose, caractérisé par la production d'α-glucosides selon l'une quelconque des revendications 1 à 15, le ou les α-glucoside (s) ainsi obtenu(s) étant ensuite mis en contact avec un acide gras au moins et une préparation enzymatique ayant une activité de lipase, suivi par la récupération des esters ainsi obtenus.

17. Procédé selon la revendication 16 caractérisé en ce que la mise en contact de l'α-glucoside avec le ou les acide (s) gras et la lipase s'effectue à une température à laquelle le ou les acide(s) gras est (sont) liquide(s), le milieu réactionnel étant dépourvu de solvant.

18. Procédé selon la revendication 17 caractérisé en ce que l'α-glucoside est un α-alkylglucoside ayant, de préférence, entre 1 et 5 atomes de carbone, et en ce que l'acide gras possède entre 8 et 20 atomes de carbone, de préférence entre 8 et 16.

19. Procédé de traitement de l'amidon natif, de farines de céréales ou de maltodextrines, caractérisé par la mise en contact de ces substrats avec un alcool dont le(s) seul(s) groupe(s) fonctionnel(s) est(sont) un(des) groupe(s) hydroxyl, en présence d'une préparation enzymatique purifiée ayant une activité d'α-tranglucosylation et dépourvue d'activité β-glucosidase.

**Patentansprüche**

1. Verfahren zur enzymatischen Herstellung von $\alpha$-Glucosiden, gekennzeichnet durch das Inkontaktbringen mindestens eines Alkohols, dessen einzige funktionelle Gruppe(n) (eine) Hydroxylgruppe(n) ist (sind), mit Stärke, Maltodextrinen oder Maltose, die in einer Konzentration von mindestens etwa 100 g/l vorliegen, in Gegenwart einer gereinigten Enzympräparation, die eine $\alpha$-Transglucosylierungs-Aktivität aufweist, aber keine $\beta$-Glucosidase-Aktivität.

2. Verfahren nach Anspruch 1, dadurch gekennzeichnet, daß die Enzympräparation aus einem Enzym besteht, das eine $\alpha$-Transglucosylierungs-Aktivität aufweist, oder aus mehreren Enzymen, von denen jedes eine $\alpha$-Transglucosylierungs-Eigenschaft hat oder, deren enzymatische Aktivität zusammengenommen der Enzympräparation eine $\alpha$-Transglucosylierungs-Aktivität verleiht.

3. Verfahren nach einem der Ansprüche 1 oder 2, dadurch gekennzeichnet, daß das oder die Enzyme $\alpha$-Transglucosidasen sind, vorzugsweise solche, die von einem Pilz, wie Talaromyces duponti, Aspergillus niger, Aspergillus oryzae oder Aspergillus batatae, stammen.

4. Verfahren nach einem der Ansprüche 1 bis 3, dadurch gekennzeichet, daß die Stärke in Form nativer Stärke vorliegt, und dadurch, daß die Aktivität der Enzympräparation, die es ermöglicht, die $\alpha$-Transglucosylierung durchzuführen, verbunden ist mit der Aktivität einer Hydrolase, wie einer Endoamylase, z.B. der $\alpha$-Amylase.

5. Verfahren nach Anspruch 4, dadurch gekennzeichnet, daß die beiden enzymatischen Reaktionen gleichzeitig ablaufen, z.B. durch die gleichzeitige Verwendung einer Endoamylase und einer $\alpha$-Transglucosidase.

6. Verfahren nach Anspruch 4 oder 5, dadurch gekennzeichnet, daß die native Stärke von Getreiden wie Weizen, Mais, Gerste, Hafer, Reis, Roggen, Buchweizen, Sorghum und Triticale stammt oder von Knollen, wie Kartoffel oder Maniok, oder von Leguminosen, wie Erbsen oder Saubohnen, oder von beliebigen anderen Pflanzen.

7. Verfahren nach Anspruch 6, dadurch gekennzeichnet, daß die Getreide in Form ganzer Körner vorliegen oder als Teile von Körnern, die z.B. aus einer enzymatischen, chemischen, thermischen oder mechanischen Behandlung des Korns resultieren, oder auch als irgendein Produkt, das als Ergebnis der Feinzerkleinerung des Korns entsteht, wie Mehl oder Grieß.

8. Verfahren nach einem der Ansprüche 1 bis 3, dadurch gekennzeichnet, daß die Stärke in Form löslicher Stärke vorliegt, beispielsweise als Resultat einer sauren oder enzymatischen Prähydrolyse.

9. Verfahren nach einem der vorangehenden Ansprüche, dadurch gekennzeichnet, daß der Alkohol ein Alkanol, ein Ethylenalkohol, ein Acetylalkohol, ein cyclischer Alkohol oder ein Phenol ist.

10. Verfahren nach Anspruch 9, dadurch gekennzeichnet, daß der Alkohol ein einfacher Alkohol oder ein Polyol ist, vorzugsweise ein Diol.

11. Verfahren nach einem der Ansprüche 9 oder 10, dadurch gekennzeichnet, das der Alkohol 1 bis 6 C-Atome enthält, mindestens teilweise in Wasser löslich ist und vorzugeweise eine Löslichkeit von mindestens 2,7 % (Vol./Vol.) bei 20°C aufweist.

12. Verfahren nach einem der Ansprüche 9 bis 11, dadurch gekennzeichnet, daß der Alkohol aus der folgenden Gruppe ausgewählt ist: Isopropanol, n-Butanol, Isobutanol, tert-Butanol, Isopentanol, Hexandiol, Buten-1-ol-3, Butin-1-ol-3 oder einem Gemisch von mindestens zwei dieser Alkolhole.

13. Verfahren nach Anspruch 9, dadurch gekennzeichnet, daß der Alkohol ein Alkanol mit 1 bis 5, und insbesondere 4, Kohlenstoffatomen ist, so daß das Produkt des Verfahrens ein $\alpha$-Alkylglucosid, insbesondere das $\alpha$-Butylglucosid, ist.

14. Verfahren nach Anspruch 9, dadurch gekennzeichnet, daß der Alkohol ein primärer aliphatischer Alkohol ist, z.B. ein Fettalkohol, der zwischen 12 und 18 Kohlenstoffatome enthält.

15. Verfahren nach einem der Ansprüche 1 bis 14, dadurch gekennzeichnet, daß es zwischen 20 und 70°C, vorzugsweise zwischen 20°C und 50°C, durchgeführt wird und bei einem pH zwischen 3 und 7, vorzugsweise zwischen 4 und 6.

**16.** Verfahren zur enzymatischen Herstellung von α-Glucosidestern ausgehend von Stärke, Maltodextrinen oder Maltose, gekennzeichnet durch die Herstellung von α-Glucosiden nach einem der Ansprüche 1 bis 15 und das Inkontaktbringen (des) der so erhaltenen α-Glucoside(s) mit zumindestens einer Fettsäure und einer Enzympräparation mit Lipaseaktivität, gefolgt von der Gewinnung der so erhaltenen Ester.

**17.** Verfahren nach Anspruch 16, dadurch gekennzeichnet, daß das Inkontaktbringen des α-Glucosids mit der oder den Fettsäure(n) und der Lipase bei einer Temperatur stattfindet, bei der die Fettsäure(n) flüssig ist (sind), wobei das Reaktionsmilieu kein Lösungsmittel enthält.

**18.** Verfahren nach Anspruch 17, dadurch gekennzeichnet, daß das α-Glucosid ein α-Alkylglucosid mit vorzugsweise zwischen 1 und 5 Kohlenstoffatomen ist, und dadurch, daß die Fettsäure zwischen 8 und 20 Kohlenstoffatome enthält, vorzugsweise zwischen 8 und 16.

**19.** Verfahren zur Behandlung nativer Stärke, von Getreidemehl oder von Maltodextrinen, gekennzeichnet durch das Inkontaktbringen dieser Substrate mit einem Alkohol, dessen einzige funktionelle Gruppe(n) (eine) Hydroxylgruppe(n) ist (sind), in Gegenwart einer gereinigten Enzympräparation, die eine α-Transglucosylierungs-Aktivität aufweist, aber keine β-Glucosidase-Aktivität.

## Claims

**1.** Process for the enzymatic manufacture of α-glucosides, characterized by placing at least one alcohol, whose only functional group(s) is (are) (a) hydroxyl group(s), in contact with starch, maltodextrins or maltose, these being at a concentration of approximately at least 100 g/l, in the presence of a purified enzymatic preparation which has α-transglucosylation activity and is devoid of β-glucosidase activity.

**2.** Process according to Claim 1, characterized in that the enzymatic preparation consists of an enzyme which has α-transglucosylation activity, or consists of several enzymes such that each has an α-transglucosylation property, or such that their combined enzymatic activities impart α-transglucosylation activity to the enzymatic preparation.

**3.** Process according to either of Claims 1 and 2, characterized in that the enzyme or enzymes are α-transglucosidases, preferably obtained from a fungus such as <u>Talaromyces duponti</u>, <u>Aspergillus niger</u>, <u>Aspergillus oryzae</u> or <u>Aspergillus batatae</u>.

**4.** Process according to any one of Claims 1 to 3, characterized in that the starch is in the form of native starch and in that the action of the enzymatic preparation capable of carrying out the α-transglucosylation is combined with the action of a hydrolase such as an endo-amylase, for example α-amylase.

**5.** Process according to Claim 4, characterized in that the two enzymatic reactions proceed simultaneously, for example by the simultaneous use of an endo-amylase and an α-transglucosidase.

**6.** Process according to Claim 4 or 5, characterized in that the native starch is obtained from cereals such as wheat, corn, barley, oats, rice, rye, buckwheat, sorghum and triticale, or from tubers such as potato or cassava, or from legumes such as peas or horse beans, or from any other plant.

**7.** Process according to Claim 6, characterized in that the cereals are in the form of whole grains or grain fractions resulting, for example, from an enzymatic, chemical, thermal or mechanical treatment of the grain, or alternatively from any product obtained by milling grain such as flour or semolina.

**8.** Process according to any one of Claims 1 to 3, characterized in that the starch is in the form of soluble starch, resulting, for example, from an acidic or enzymatic prehydrolysis.

**9.** Process according to any one of the preceding claims, characterized in that the alcohol is an alkanol, an ethylenic alcohol, an acetylenic alcohol, a cyclic alcohol or a phenol.

10. Process according to Claim 9, characterized in that the alcohol is a simple alcohol or a polyol, preferably a diol.

11. Process according to either of Claims 9 and 10, characterized in that the alcohol contains from 1 to 6 carbon atoms, is at least partially soluble in water and, preferably, has a solubility of at least 2.7% (v/v) at 20°C.

12. Process according to any one of Claims 9 to 11, characterized in that the alcohol is chosen from the following group: isopropanol, n-butanol, isobutanol, tert-butanol, isopentanol, hexanediol, 1-buten-3-ol, 1-butyn-3-ol or a mixture of at least two of these alcohols.

13. Process according to Claim 9, characterized in that the alcohol is an alkanol containing from 1 to 5, and in particular 4, carbon atoms, the product of the process then being an α-alkylglucoside, in particular α-butylglucoside.

14. Process according to Claim 9, characterized in that the alcohol is a primary aliphatic alcohol, for example a fatty alcohol having between 12 and 18 carbon atoms.

15. Process according to any one of Claims 1 to 14, characterized in that it is carried out at between 20 and 70°C, preferably between 20°C and 50°C, and at a pH of between 3 and 7, preferably of between 4 and 6.

16. Process for the enzymatic manufacture of α-glucoside esters from starch, maltodextrins or maltose, characterized by the production of α-glucosides according to any one of Claims 1 to 15, the α-glucosides(s) thus obtained then being placed in contact with at least one fatty acid and an enzymatic preparation having lipase activity, followed by recovery of the esters thus obtained.

17. Process according to Claim 16, characterized in that the α-glucoside is placed in contact with the fatty acid(s) and the lipase at a temperature at which the fatty acid(s) is (are) liquid, the reaction medium being free of solvent.

18. Process according to Claim 17, characterized in that the α-glucoside is an α-alkylglucoside preferably having between 1 and 5 carbon atoms, and in that the fatty acid contains between 8 and 20 carbon atoms, preferably between 8 and 16.

19. Process for the treatment of native starch, cereal flours or maltodextrins, characterized by the placing in contact of these substrates with an alcohol whose only functional group(s) is(are) (a) hydroxyl group(s), in the presence of a purified enzymatic preparation which has α-transglucosylation activity and which is devoid of β-glucosidase activity.

## FIGURE 1

# FIGURE 2A

## FIGURE 2B

**FIGURE 2C**

**FIGURE 3A**

## FIGURE 3B